# EUROPEAN PATENT SPECIFICATION

(11) **EP 0 889 994 B1**
(45) Date of publication and mention of the grant of the patent: **12.06.2002**
(21) Application number: 97915813.6
(22) Date of filing: 24.03.1997
(51) Int. Cl.: D21H 17/63, D21H 21/22

(54) **ABSORBENT MATERIAL AND PRODUCTION THEREOF**
ABSORBIERENDES MATERIAL UND HERSTELLUNGSVERFAHREN
MATERIAU ABSORBANT ET SON PROCEDE DE PRODUCTION

(30) Priority: 25.03.1996 SE 9601136
(43) Date of publication of application: 13.01.1999
(73) Proprietor: Eka Chemicals AB, 445 80 Bohus (SE)
(72) Inventor: THEBRIN, Ingemar, 444 41 Stenungsund (SE); WAHLEN, Svante, S-444 42 Stenungsund (SE); LINDGREN, Erik, S-445 34 Bohus (SE); MALMBORG, Kerstin, S-440 74 Hjälteby (SE)
(74) Representative: Jönsson, Christer
(86) International application number: SE9700510
(87) International publication number: WO9736047

(56) References cited:
- EP-A- 0 540 076
- WO-A-91/11977
- WO-A-97/36046
- US-A- 4 826 497

## Description

The present invention relates to a method for production of hygienic paper having improved sorptive capacity, to hygienic paper obtainable by such a method, and to absorbent articles comprising such paper.

In the present context the concept of "sorptive capacity" aims at the rate by which the hygienic paper takes up liquids such as water or aqueous solutions, including body fluids such as urine, blood and menstrual fluids, or to the liquid-retaining capacity of the hygienic paper, or to both of these characteristics. The sorptive mechanism may be adsorption or absorption, or a combination thereof.

As stated in Ullmann's encyclopedia of industrial chemistry, Vol. A 18, page 663, 1991, the term hygienic paper encompasses cellulose wadding, soft tissue, and crepe paper, all useful within the household or sanitary fields. It is well known that the rate of liquid uptake and liquid-holding capacity of hygienic paper are important when used in these fields. This is particularly valid with regard to absorbent articles such as catamenial devices (e.g. sanitary napkins, pantiliners, tampons etc.), diapers, bandages, adult incontinence garments, and the like; good liquid uptake and liquid-holding capacity are obvious prerequisites for the function of such articles. However, when used, an article of this kind is constantly subjected to pressure imposed by the weight and the movements of the bearer, and thus it is important that the liquid-holding capacity is high enough to retain the absorbed liquid also under pressure. Furthermore, in order to give good comfort to the bearer, the article should provide a feeling of dryness, meaning that any rewetting from the article to the skin of the bearer should by avoided, raising the requirements with regard to liquid-holding capacity even higher. Conventionally, certain polymeric materials forming hydrogels in contact with water, known as "superabsorbents", have been utilised to enhance the sorptive capacity of such articles; however, although this capacity of the article is enhanced, as the liquid is bound to superabsorbent particles, the sorptive capacity of the cellulosic fibres making up the hygienic paper itself is not in fact enhanced by the use of such superabsorbents.

EP-A-0 540 076 discloses a sized paper and its preparation. The paper contains a high-ratio hydrophobic zeolite for adsorbing undesired odours. The addition of the zeolite is carried out before forming and dewatering the suspension of lignocellulose-containing fibres when the dry matter content of the fibres is low which in turn results in a product having improved water-repellent capacity.

Another field in which the sorptive capacity of cellulosic material is important is that of laminates, particularly so-called decorative laminates, such as described in US-A-3,373,071. Such laminates conventionally comprise at least three layers: a wear surface layer, a print or pattern layer beneath the wear surface layer, and a core layer supporting the wear surface and print layers. All layers consist of paper sheet impregnated with thermosetting resin. The wear surface layer usually consists of translucent paper impregnated with melamine resin. The impregnation is carried out by submerging the paper in an aqueous solution of the resin. Obviously the sorptive capacity of the paper is a critical parameter for the efficiency of the impregnation.

The problem to be solved by the present invention is thus to provide a method for production of hygienic paper having improved sorptive capacity.

This problem is solved by the method defined by the appended claims. More specifically, the present invention relates to a method for production of hygienic paper having improved sorptive capacity for taking up liquids such as water or aqueous solutions, which paper comprises cellulosic fibres, in which the fibres are treated, in the presence of water, with a hydrophobic substance having a specific surface area of at least 50 m²/g, by bringing the substance and the fibres together when the fibres have a dry content of at least 20%.

It is believed that the reason behind the improved sorption characteristics imposed by the hydrophobic substances of the stated kind may be that the substances absorbate surface active substances naturally occurring in or on the cellulosic fibres.

To "treat" the fibres with a hydrophobic substance means that particles of the substance are brought into the proximity of, or in contact with the fibres when the dry matter content of the fibres is at least about 20%, suitably at least about 25%, and most preferably at least about 30%. This means that the present method may be carried out in a process for production of hygienic paper once the drainage or dewatering of the web is completed. In this application "web" also comprises the concept of "sheet". Preferably, the particles are kept in the proximity of, or in contact with, the fibres for at least about 10 seconds, suitably at least about 5 seconds, and particularly at least about 2 seconds.

The specific surface area of the hydrophobic substance particles, determined according to standard method DIN 66131 modified as described below, is at least about 50 m²/g, preferably at least about 100 m²/g, and most preferably at least about 200 m²/g. For activated carbon the specific surface area is about 1000 m²/g, for zeolite of type ZSM-5 and type A it is about 500 m²/g, whereas zeolite X and Y both have a specific surface areas of about 800 m²/g.

The hydrophobic substance may be any substance that is substantially insoluble in water, preferably having a solubility not higher than about 1 g/100 g of water, suitably not higher than about 0.1 g/100 g of water, as long as it has a specific surface area as indicated above. Exemplary of useful hydrophobic substances are activated carbon, hydrophobic zeolites, and polytetrafluoroethylene (i.e. Teflon). The substances are preferably porous. In a particularly preferred embodiment the hydrophobic substance is activated carbon or a zeolite having a hydrophobicity of below about 0.99 percent, preferably below about 0.90 percent, and suitably below about 0,70 percent by weight residual butanol as determined by the Residual Butanol Test described below. Especially preferred zeolites are those having a molar relation SiO₂/Al₂O₃ of about 5.

It is preferred that the hydrophobic substance is comprised in an aqueous mixture, usually as a dispersion or a slurry. The mixture is applied on the web, conveniently by spraying or showering. The amount of substance added is suitably about 0.1 - 10 kg/metric ton dry pulp, preferably about 0.5 - 5 kg/ton. Optionally the treatment may be carried out by pouring the substance, preferably comprised in an aqueous mixture, onto the web, or by submerging the web into such a mixture.

The treatment may be carried out in the presence of a retention agent to ensure that a sufficient amount of the substance particles are kept in contact with, or in the proximity of the fibres long enough to give the desired effect. Exemplary of preferred retention agents are polysaccharides, such as starch, cellulose derivatives, xanthan gum and guar gum, and synthetically produced homopolymers, such as polyacryl amide (PAM), polyamide amine (PAA), polydiallyl dimethyl ammonium chloride (polyDADMAC), polyethylene imine (PE1) and polyethylene oxide (PEO), or copolymers thereof.

Although the substance may remain in the hygienic paper after the treatment, this is not considered to be essential to the invention. In fact, even if less than about 65%, say less than 30%, or even as little as about 1% of the substance used in the treatment is present in the hygienic paper after the treatment, the sorption effect is quite noticeably enhanced when compared to untreated paper. In some instances it may however be advantageous to let a substantial amount of substance remain in the paper, for instance when the substance is a hydrophobic zeolite providing such odour eliminating characteristics to absorbent articles, e.g. diapers and catamenial devices, as disclosed in US-A-4,826,497 and WO 91/11977; it should, however, be noted that in those documents the zeolite is not used for treatment of the fibres in the presence of water.

The present invention also relates to hygienic paper obtainable by the present method, and to absorbent articles such as diapers, sanitary napkins, pantiliners, tampons, bandages, adult incontinence garments, toilet paper, handkerchiefs, kitchen towels, hand towels, face towels, serviettes, and similar articles comprising such paper.

Hygienic paper may be produced by any suitable paper-making process. Soft tissue and cellulose wadding qualities are usually manufactured by means of a so-called Yankee machine, i.e. a paper machine in which the drying is achieved substantially on a Yankee cylinder or Yankee dryer, which is a drying cylinder with a large diameter and a polished surface which is largely responsible for the drying of the paper web. Usually the wet web is adhered to the Yankee cylinder at 20-35% dryness with one or two pressure rolls; in this context, the present invention may be carried out by means of spray showers situated before the first pressure roll.

The fibres making up the pulp sheet are usually obtained by disintegrating wood, conventionally in the form of chips, into fibres or bundles of fibres; in the present context the concept of "bundles of fibres" is regarded to be equivalent to the concept of "fibres". The separated fibres may be obtained by means of any pulp-making method known to a skilled person, e.g. by a method for production of mechanical pulp (MP), stone groundwood pulp (SGW), pressure groundwood pulp (PGW), refiner mechanical pulp (RMP), thermomechanical pulp (TMP), chemi-mechanical pulp (CMP), or chemi-thermomechanical pulp (CTMP), although the preferred pulps are chemical pulps such as, for instance, sulphate and sulphite pulps. However, the cellulosic fibres may also be cotton fibres. Another plausible source of fibres is recycled fibres from wastepaper.

The present invention is illustrated in more detail below by means of examples. Unless otherwise stated the parts and percentages below are given by weight. In the Examples below, the absorbent material obtained is tested with respect to rewetting and/or with respect to the uptake rate. The test method for determination of the uptake rate was SCAN-P 61-87, in which test a sample consisting of a bundle of six tissue sheets is placed in an apparatus especially designed for the test method, which is equipped with electrodes for detection of penetrating water in the machine direction, in the cross direction, and in the direction perpendicular to the surface of the tissue, respectively. The time elapsing from application of a certain amount of water on the tissue until the electrodes detect the penetration is determined for the respective directions. The shorter time required to penetrate the material, the higher is the uptake rate of the material.

Example 1-2: Tissue made from a pulp composition containing 40% sulphate pulp of eucalyptus wood, 36% sulphate pulp of pine, and 24% sulphite pulp of spruce was used in this Example. The basis weight of the tissue was 18.6 g/m². An aqueous solution containing 0.2 % of a zeolite was sprayed onto one sample; said zeolite was of Y type and had a hydrophobicity of 0.28 percent by weight residual butanol as determined by the Residual Butanol Test, and a SiO₂/Al₂O₃ ratio of 29. The specific surface area was measured to be about 800 m²/g by a method based on DIN 66131 (the so-called BET method), in which the area is determined at one spot at a relative pressure P/P₀ of 0.03. The amount of zeolite added to the sample corresponded to 3 kg zeolite/metric ton of dry tissue. The tissue sample was allowed to dry and was then tested for determination of the uptake rates. Example 1 is a comparison example with no zeolite added. The results are set forth in Table I below.

**Table I**

| Ex | Uptake rate in machine direction, s | Uptake rate in cross direction, s | Uptake rate in direction perpendicular to tissue surface, s |
|---|---|---|---|
| 1 | 2.88 | 0.81 | 0.06 |
| 2 | 2.42 | 0.68 | 0.04 |

Tissue treated according to the present invention thus evidently has a faster uptake rate than untreated tissue.

## Claims

1. A method for production of hygienic paper having improved sorptive capacity for taking up liquids such as water or aqueous solutions , which paper comprises cellulosic fibres, **characterised in that** the fibres are treated, in the presence of water, with a hydrophobic substance having a specific surface area of at least 50 m²/g, by bringing the substance and the fibres together when the fibres have a dry content of at least 20%.

2. A method according to claim 1, **characterised in that** a web is formed by draining an aqueous suspension of cellulosic fibres and then pressing and/or drying the web, and that the web is treated with the substance after the draining.

3. A method according to claim 1 or 2, **characterised in that** the fibres are sprayed or showered with a mixture comprising the substance and water.

4. A method according to anyone of claims 1-3, **characterised in that** the substance is activated carbon or a zeolite having a hydrophobicity of below about 0.99 percent by weight residual butanol as determined by the Residual Butanol Test.

5. Hygienic paper obtainable by a method according to anyone of claims 1-4.

6. Hygienic paper according to claim 5, **characterised in that** the hygienic paper is cellulose wadding, soft tissue or crepe paper.

7. An absorbent article comprising hygienic paper according to any one of claim 5-6.

8. An absorbent article according to claim 7, **characterised in that** the article is a diaper, a sanitary napkin, a pantiliner, a tampon, a bandage, an adult incontinence garment, toilet paper, a handkerchief, a kitchen towel, a hand towel, a face towel, or a serviette.

## Patentansprüche

1. Verfahren zur Herstellung von Hygienepapier mit verbesserter Sorptionskapazität zum Aufnehmen von Flüssigkeiten wie Wasser oder wässrigen Lösungen, wobei das Papier Zellstofffasern enthält, **dadurch gekennzeichnet, dass** die Fasern in Gegenwart von Wasser mit einem hydrophoben Stoff, der eine spezifische Oberfläche von mindestens 50 m²/g aufweist, behandelt wird, indem der Stoff und die Fasern zusammen gebracht werden, wenn die Fasern einen Trockengehalt von mindestens 20 % aufweisen.

2. Verfahren nach Anspruch 1, **dadurch gekennzeichnet, dass** durch Entwässern einer wässrigen Suspension von Zellstofffasern ein Vlies erzeugt wird, das Vlies danach gepresst und/oder getrocknet wird, und dass das Vlies nach dem Entwässern mit dem Stoff behandelt wird.

3. Verfahren nach einem der Ansprüche 1 oder 2, **dadurch gekennzeichnet, dass** die Fasern mit einem den Stoff und Wasser umfassenden Gemisch besprüht oder besprengt werden.

4. Verfahren nach einem der Ansprüche 1 bis 3, **dadurch gekennzeichnet, dass** der Stoff Aktivkohle oder ein Zeolith ist, der eine Hydrophobizität von weniger als ungefähr 0,99 Gewichtsprozent Butanol-Rückstand, bestimmt durch die Prüfung auf Butanol-Rückstände, aufweist.

5. Hygienepapier, erhältlich durch ein Verfahren nach einem der Ansprüche 1 bis 4.

6. Hygienepapier nach Anspruch 5, **dadurch gekennzeichnet, dass** das Hygienepapier Zellstoffwatte, weiches Seidenpapier oder Krepppapier ist.

7. Absorbierender Artikel, enthaltend Hygienepapier nach einem der Ansprüche 5 bis 6.

8. Absorbierender Artikel nach Anspruch 7, **dadurch gekennzeichnet, dass** der Artikel eine Windel, eine Monatsbinde, eine Slipeinlage, ein Tampon, ein Verband, eine Inkontinenzbekleidung für Erwachsene, Toilettenpapier, ein Taschentuch, ein Küchentuch, ein Handtuch, ein Kosmetiktuch oder eine Serviette ist.

## Revendications

1. Procédé de production de papier hygiénique ayant une capacité de sorption améliorée pour la fixation de liquides tels que l'eau et les solutions aqueuses, ledit papier comprenant des fibres cellulosiques, procédé **caractérisé en ce que** l'on traite les fibres, en présence d'eau, avec une substance hydrophobe ayant une surface spécifique d'au moins 50 m2/g, en mettant en présence la substance et les fibres lorsque les fibres ont une teneur en matière sèche d'au moins 20 %.

2. Procédé selon la revendication 1, **caractérisé en ce que** l'on forme une bande par égouttage d'une suspension aqueuse de fibres cellulosiques, puis compression et/ou séchage de la bande, et **en ce que** l'on traite la bande avec la substance après l'égouttage.

3. Procédé selon la revendication 1 ou 2, **caractérisé en ce que** l'on pulvérise sur les fibres un mélange comprenant la substance et de l'eau ou bien on les arrose avec un tel mélange.

4. Procédé selon l'une quelconque des revendications 1 à 3, **caractérisé en ce que** la substance est du charbon actif ou une zéolite ayant un degré d'hydrophobie, déterminé par le test au butanol résiduaire, correspondant à moins d'environ 0,99 % en poids de butanol résiduaire.

5. Papier hygiénique que l'on peut obtenir par le procédé selon l'une quelconque des revendications 1 à 4.

6. Papier hygiénique selon la revendication 5, **caractérisé en ce que** ce papier hygiénique est de l'ouate de cellulose, du tissu doux ou du papier crêpe.

7. Article absorbant comprenant du papier hygiénique selon l'une quelconque des revendications 5 et 6.

8. Article absorbant selon la revendication 7, **caractérisé en ce qu'**il s'agit d'une couche, d'une serviette hygiénique, d'un protège-slip, d'un tampon, d'un bandage, d'un vêtement pour l'incontinence des adultes, de papier de toilette, d'un mouchoir, d'une serviette pour la cuisine, d'une serviette de toilette pour les mains, d'une serviette de toilette pour la face ou d'une serviette de table.
